# FASCICULE DE BREVET EUROPEEN

(11) **EP 3 353 191 B1**
(45) Date de publication et mention de la délivrance du brevet: **24.05.2023**
(21) Numéro de dépôt: 16794681.3
(22) Date de dépôt: 23.09.2016
(51) Int. Cl.: C07K 7/64, A61K 38/00

(54) **POLYPEPTIDES CYCLIQUES, LEUR PROCEDE D'OBTENTION ET LEUR APPLICATION EN THERAPEUTIQUE**
CYCLISCHE POLYPEPTIDE, VERFAHREN ZUR HERSTELLUNG BESAGTER POLYPEPTIDE UND THERAPEUTISCHE VERWENDUNG DAVON
CYCLIC POLYPEPTIDES, METHOD FOR OBTAINING SAID POLYPEPTIDES AND THE THERAPEUTIC USE THEREOF

(30) Priorité: 25.09.2015 FR 1559084
(43) Date de publication de la demande: 01.08.2018
(73) Titulaire: Axoltis Pharma, 63100 Clermont-Ferrand (FR)
(72) Inventeur: BRIDON, Dominique, San Francisco, CA 94114 (US); GOBRON, Stéphane, 63111 Dallet (FR)
(74) Mandataire: Lavoix
(86) Numéro de dépôt international: PCT/FR2016/052422
(87) Numéro de publication internationale: WO 2017/051135

(56) Documents cités:
- EP-A2- 0 478 101
- WO-A1-2009/027350
- MAULIK TRIVEDI ET AL: "The Role of Thiols and Disulfides on Protein Stability", CURRENT PROTEIN AND PEPTIDE SCIENCE, vol. 10, no. 6, 1 décembre 2009 (2009-12-01), pages 614-625, XP055234745, NL ISSN: 1389-2037, DOI: 10.2174/138920309789630534

## Description

L'invention a pour objet de nouveaux polypeptides cycliques, les compositions pharmaceutiques les comprenant, et leur utilisation comme médicaments, notamment leur utilisation dans le traitement des maladies neurodégénératives et la régénération des cellules du système nerveux central et périphérique, et le procédé d'obtention de ces polypeptides cycliques.

Les polypeptides de l'invention ont une séquence d'acides aminés dérivant de l'un des domaines consensus conservés de la SCO-spondine appelé répétition thrombospondine de type 1 ou TSR.

Il est bien connu que différents peptides synthétiques déduits de la structure de la thrombospondine ont des actions intéressantes sur différents types cellulaires, notamment ils inhibent les tumeurs chez les mammifères, influencent la thrombolyse et l'angiogenèse et peuvent être des modulateurs du complément ou bien assurer la promotion de l'attachement cellulaire [Sipes JM et al., J Cell Biol, 121, 469-77, 1993; Rusnati M et al. Pharmaceuticals 3, 1241-1278, 2010; Lopez-Dee Z et al., Mediators of Inflammation, volume 2011, Article ID 296069, 10 pages, 2011].

Les caractéristiques générales de la SCO-spondine sont décrites notamment dans l'article de Meiniel et al., Microsc Res Tech. 2, 484-95, 2001 et l'article de Gobron et al. Glia 32, 177-91, 2000.

L'application d'un polypeptide de séquence d'acides aminés :
W-S-G-W-S-S-C-S-R-S-C-G, [SEQ ID NO : 58] correspondant à la séquence d'acides aminés la plus représentative d'un des motifs TSR de la SCO-spondine, entraine chez les cellules B 104 issues d'un neuroblastome de rat une différenciation cellulaire induisant une pousse neuritique et une agrégation cellulaire [F. El-Bitar et al., Cell Tissue Res., vol. 304, p. 361-369, 2001]. Ce polypeptide ne comporte pas de pont disulfure entre les deux Cystéines.

Ce polypeptide ainsi que les polypeptides sous formes réduites de formule SEQ ID NO : 70 et en particulier de formule SEQ ID NO : 57 sont décrits et revendiqués dans les demandes de brevet international WO 1999/03890 (correspondant au brevet américain US 6 995 140) et WO2009027350 ou peuvent être préparés à partir de la description de ces brevets et des méthodes connues de l'homme de métier.

Cependant, la stabilité du polypeptide W-S-G-W-S-S-C-S-R-S-C-G [SEQ ID NO : 58] diminue en solution aqueuse avec le temps. Il serait donc nécessaire que ce polypeptide soit préparé extemporanément lors des traitements, ce qui pourrait en compliquer l'administration, limitant ainsi les possibilités d'effectuer des traitements par injection directe, ou par exemple au moyen de pompes pour la délivrance progressive du médicament aux patients. D'une manière générale, cette faible stabilité présenterait un certain inconvénient pour le développement de concepts thérapeutiques basés sur ce polypeptide.

La demande de brevet WO 2008/090 285 décrit des analogues peptido-mimétiques du polypeptide W-S-G-W-S-S-C-S-R-S-C-G [SEQ ID NO : 58]. Cette approche a toutefois deux inconvénients : ces polypeptides contiennent des acides aminés non-naturels susceptibles d'engendrer des phénomènes d'immunogénicité, et une augmentation sensible de coûts de production, les acides aminés non naturels étant beaucoup plus coûteux que les acides aminés naturels, ce qui diminue leur intérêt commercial.

L'un des buts de l'invention est de fournir des composés peptidiques stables et solubles dans des solutions compatibles notamment avec les administrations intrathécales tout en conservant ou améliorant les propriétés du polypeptide de séquence W-S-G-W-S-S-C-S-R-S-C-G [SEQ ID NO : 58].

Il est en effet particulièrement important pour certaines indications cliniques recherchées de développer une forme plus soluble du polypeptide de séquence d'acides aminés W-S-G-W-S-S-C-S-R-S-C-G [SEQ ID NO : 58] décrit dans la demande de brevet international WO 1999/03890.

En effet, pour les injections intrathécales (IT), le choix des solutions utilisées pour administrer le composé est très limité. En pratique, il n'y a que trois solutions utilisées, le liquide physiologique, le liquide cérébro-rachidien artificiel et les solutions de glucose 5%. D'autres solutions connues de l'homme du métier peuvent être utilisées si d'autres voies d'administration telles que les voies intraventriculaire, épidurale, intrarachidienne, intraparenchymateuse, intraveineuse, intraluminale, intravitréale, transphénoïdale ou topique sont utilisées. Par ailleurs, des compositions pharmaceutiques pourront être produites sous une forme quelconque, notamment liquide, en particulier des préparations injectables sous forme de solutions, suspensions ou émulsions, des préparations pour perfusion, ou solide et comporter tout type de support, en particulier gels, biopolymères ou biomatériaux ou tout dispositif médical, comme les implants ou les pompes implantables par exemple, permettant une libération contrôlée ou bien un système de vectorisation.

Les solutions aqueuses peuvent être réalisées en utilisant divers solvants compatibles avec la voie d'administration, idéalement un solvant isotonique comme le chlorure de sodium à 0,9% ou de glucose à 5%, mais le solvant pourra également être l'eau, une solution saline, des tampons phosphate, citrate ou acétate ou tout autre. Eventuellement, la préparation pourra également contenir, en combinaison ou non, tout excipient, adjuvant ou additif tels que le propylène glycol, glycérol, polyéthylène glycol ou tout agent tensioactif, mouillant, viscosifiant, chelatant, isotonisant, antioxydant ou stabilisant.

Les préparations sous forme de solution pourront être administrées par injection bolus ou par perfusion.

Dans le cas du polypeptide de séquence d'acides aminés : W-S-G-W-S-S-C-S-R-S-C-G, [SEQ ID NO : 58], la solubilité dans le liquide physiologique est basse, de l'ordre de 1-2 mg/mL, et donc rend plus difficile l'obtention des doses cliniques maximum souhaitées et qui sont de l'ordre de 10 mg/mL. La solubilité est particulièrement importante pour les injections IT car il y a une limitation du volume d'injection, limite qui est bien plus grande que celle pour les injections périphériques, et une limitation dans les solvants et solutés utilisables par exemple. L'utilisation d'une solution de glucose 5% améliore la solubilité du polypeptide de séquence d'acides aminés : W-S-G-W-S-S-C-S-R-S-C-G [SEQ ID NO : 58] en la portant aux environs de 10 mg/mL, cette solution restant une solution isotonique et compatible avec une injection IT.

Toutefois, la présence de glucose en contact avec le polypeptide est connue pour former des bases de Schiff, venant de la réaction de la forme aldéhyde du glucose avec les amines primaires des polypeptides selon le schéma réactionnel :

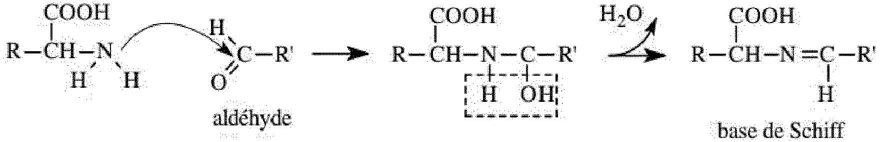

Ces bases de Schiff pourraient théoriquement compliquer la préparation et la mise en oeuvre des compositions pharmaceutiques. Cependant dans le cas des présents polypeptides ces bases sont réversibles in vivo.

Il est donc très important de développer une forme plus soluble du polypeptide de séquence d'acides aminés : W-S-G-W-S-S-C-S-R-S-C-G, [SEQ ID NO : 58] dans un solvant autre que le glucose 5% et préférablement dans le liquide physiologique ou le liquide cérébro-rachidien artificiel.

Un autre aspect de l'invention est de fournir des composés peptidiques efficaces dans le traitement des maladies neurodégénératives ou des traumatismes nerveux dans lesquels la régénération des cellules nerveuses est nécessaire ainsi que des compositions pharmaceutiques les comprenant.

Un autre aspect de l'invention est de fournir un procédé d'obtention de ces composés peptidiques.

La présente invention est telle que définie aux revendications attachées.

Au sens de la présente invention, on entend par « acides aminés » ou « aminoacides » aussi bien les acides aminés naturels que les acides aminés non naturels.

Par « acides aminés naturels », on entend les acides aminés sous forme L que l'on peut trouver dans les protéines naturelles, c'est-à-dire l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la proline, la sérine, la thréonine, le tryptophane, la tyrosine et la valine.

Par « acide aminé non naturel », on entend les acides aminés précédents sous leur forme D, ainsi que les formes homo de certains acides aminés comme l'arginine, la lysine, la phénylalanine et la sérine ou les formes nor de la leucine ou de la valine.

La nomenclature utilisée pour décrire les séquences peptidiques est la nomenclature internationale utilisant le code à une lettre et où l'extrémité amino-terminale est présentée à gauche et l'extrémité carboxy-terminale est présentée à droite.

Les tirets « - » représentent des liaisons peptidiques communes liant les acides aminés des séquences.

Au sens de la présente invention, on entend par polypeptide tout polymère d'acides aminés reliés entre eux par des liaisons peptidiques, quelle que soit sa longueur. Ainsi, au sens de la présente invention, le terme polypeptide englobe également les peptides et les protéines.

L'invention concerne un polypeptide comprenant la séquence d'acides aminés suivante :
W-S-G-W-S-S-C-S-R-S-C-G (SEQ ID NO : 2) dans laquelle les deux cystéines forment un pont disulfure.

Le produit SEQ ID NO : 2 a donc pour structure :

W-S-G-W-S-S-C-S-R-S-C-G
S——S

Que l'on peut également représenter, avec la nomenclature internationale utilisant le code à trois lettres, selon la formule :

Trp-Ser-Gly-Trp-Ser-Ser-Cys-Ser-Arg-Ser-Cys-Gly
S S

Ou selon la formule:

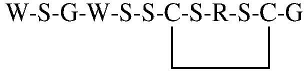
W-S-G-W-S-S-C-S-R-S-C-G

De manière surprenante, les inventeurs ont trouvé que la formation d'un pont disulfure entre les deux cystéines présentes dans le polypeptide de séquence d'acides aminés W-S-G-W-S-S-C-S-R-S-C-G [SEQ ID NO : 2], permettait non seulement de conserver l'efficacité du polypeptide pour induire une augmentation de la pousse neuritique et une augmentation des contacts synaptiques, mais aussi d'obtenir un polypeptide présentant une meilleure stabilité et une meilleure solubilité dans des solutions compatibles avec les administrations notamment intrathécales.

Cette invention est d'autant plus surprenante que, dans la protéine SCO-spondine de laquelle est issu cette séquence peptidique, les deux cystéines présentes dans la séquence W-S-G-W-S-S-C-S-R-S-C-G [SEQ ID NO : 2], ci-dessus ne forment pas un pont disulfure entre elles mais sont au contraire engagées dans des ponts disulfure avec d'autres cystéines présentes dans la protéine.

L'expression « pratiquement dépourvu » signifie que le polypeptide selon le mode de réalisation préférentiel indiqué ci-dessus, comprend des quantités très réduites des impuretés que les inventeurs de la présente demande ont identifiées. Ces impuretés comprennent la structure réduite dans laquelle les deux groupements thiol des deux cystéines sont sous forme libre, des dimères (ou polymères) ainsi que des formes oxydées dans lesquelles les groupements thiols sont sous forme de sulfoxide ou de sulfone. L'obtention de cette forme pratiquement pure est réalisée de préférence en mettant en oeuvre le procédé décrit ci-après. Dans ces conditions, la pureté du produit obtenu est de l'ordre de 80% au minimum. Cette pureté peut atteindre 85, 90 ou même 95%.

Selon un mode de réalisation plus particulier, l'invention concerne ainsi un polypeptide consistant en la séquence d'acides aminés suivante :
W-S-G-W-S-S-C-S-R-S-C-G (SEQ ID NO : 2)
dans laquelle les deux cystéines forment un pont disulfure,
ledit polypeptide étant pratiquement dépourvu des formes réduite ou dimérique ou oligomérique dudit polypeptide ainsi que des dérivés dudit polypeptide dans lesquels les groupements thiols sont sous forme de sulfoxide ou de sulfone.

Des procédés de purification classiques, par exemple par chromatographie peuvent être utilisés pour purifier les produits comportant un pont disulfure recherchés.

Par exemple, la synthèse du composé correspondant à la séquence SEQ ID NO : 2 effectuée par les méthodes connues de l'homme du métier en absence d'albumine, produit un mélange d'environ 60 produits, parmi lesquels se trouve le produit recherché avec une pureté de 44.51% déterminée par HPLC. Plusieurs HPLC successives effectuées en utilisant un gradient 0.1% TFA/eau/acetonitrile suivies par un mélange des fractions identiques (pooling) permettent d'isoler le composé correspondant à la séquence SEQ ID NO : 2 avec une pureté finale de 99%.

Selon un mode de réalisation encore plus particulier, l'invention concerne donc un polypeptide tel que défini ci-dessus, consistant en la séquence d'acides aminés suivante :
W-S-G-W-S-S-C-S-R-S-C-G (SEQ ID NO : 2)
dans laquelle les deux cystéines forment un pont disulfure,
ledit polypeptide présentant une pureté supérieure à 80%, de préférence 85%, plus préférentiellement 90%, plus préférentiellement encore égale ou supérieure à 95%.

Selon un autre aspect, l'invention concerne un procédé d'obtention du polypeptide de séquence d'acides aminés SEQ ID NO : 2:
W-S-G-W-S-S-C-S-R-S-C-G (SEQ ID NO : 2) dans laquelle :
les deux cystéines forment un pont disulfure,
comprenant une étape de formation d'un pont disulfure en présence d'albumine dans le polypeptide de séquence SEQ ID NO : 58:
   W-S-G-W-S-S-C-S-R-S-C-G (SEQ ID NO : 58)

Les inventeurs de la présente demande ont découvert une méthode inventive et inattendue de formation d'un pont disulfure. Cette méthode consiste à utiliser l'albumine comme catalyseur oxydant afin de diminuer ou d'éviter la formation d'impuretés et aussi afin d'accélérer la cinétique de réaction, un résultat toujours favorable pour la production industrielle de peptides.

La présence d'albumine dans la réaction permet notamment d'accélérer l'oxydation du produit de départ. Dans une réaction d'oxydation, il est souvent souhaitable d'accélérer de manière douce la réaction afin d'éviter la création de produit non désirables venant d'une oxydation non contrôlée. Des résultats fournis dans la partie expérimentale montrent que la réaction en présence d'albumine permet un meilleur contrôle des produits formés (plus particulièrement du polypeptide de séquence SEQ ID NO : 2) et donc une meilleure pureté.

Plus particulièrement, l'invention concerne un procédé d'obtention du polypeptide de séquence SEQ ID NO : 2 tels que défini ci-dessus, caractérisé en ce que l'albumine et le polypeptide de séquence SEQ ID NO : 58 sont présents dans un ratio albumine:polypeptide de 1:1 à 1:100, de préférence de 1:1 à 1:10 et plus préférentiellement 1:1.

Selon un autre mode de réalisation particulier, l'invention concerne un procédé d'obtention du polypeptide de séquence SEQ ID NO : 2 tel que précédemment définis, caractérisé en ce que l'étape de formation d'un pont disulfure en présence d'albumine dans le polypeptide de séquence SEQ ID NO : 58 est réalisée à l'air ambiant.

Selon un mode de réalisation avantageux, l'invention concerne un procédé d'obtention du polypeptide de séquence SEQ ID NO : 2 tel que précédemment défini, caractérisé en ce que l'étape de formation d'un pont disulfure en présence d'albumine dans le polypeptide de séquence SEQ ID NO : 58 est réalisée sans détacher le polypeptide de séquence SEQ ID NO : 58 de la résine utilisée pour la synthèse peptidique de ces polypeptides et en ce que le polypeptide de séquence SEQ ID NO : 2 est ensuite obtenu en séparant le polypeptide de la résine après l'étape de formation du pont disulfure.

Le procédé de préparation du polypeptide de séquence SEQ ID NO : 58 est basé sur la synthèse peptidique en phase solide utilisant des acides aminés protégés par N-α-Fmoc comme synthons pour la construction du polypeptide.

Le résidu glycyle en position C-terminale est couplé à la résine MBHA comme élément du linker Fmoc-Gly-MPPA-OH. Les autres résidus d'acide aminé sont incorporés par une succession de cycles de déprotection du groupe Fmoc et de couplage d'acides aminés pour produire le polypeptide protégé lié à la résine. Après l'assemblage en phase solide du polypeptide, une réaction de clivage du polypeptide de la résine et une déprotection dudit polypeptide sont effectuées simultanément en une étape avec un mélange acide trifluoroacétique (TFA) / eau pour produire le polypeptide brut, qui est précipité à l'aide d'un mélange MTBE/hexane, puis filtré et séché.

Avant la purification, le polypeptide brut de séquence SEQ ID NO : 58 est dissous dans un mélange acétonitrile/eau/acide acétique (AcOH). La purification est réalisée par une chromatographie en phase inverse préparative en utilisant d'abord un éluent d'acide trifluoroacétique (TFA) puis un éluant d'acétate. Le polypeptide purifié obtenu (sous forme de sel d'acétate) et en solution est dilué avec de l'eau et concentré. Après addition de 5% d'acétonitrile, la solution obtenue est filtrée et lyophilisée pour donner le polypeptide de séquence SEQ ID NO : 58 sous sa forme de substance médicamenteuse.

Cette méthode présente plusieurs avantages. Les travaux effectués directement sur résine sont mieux contrôlés donc plus propres. Ils produisent moins d'impuretés et évitent plusieurs étapes intermédiaires (dont la purification complexe des polypeptides de séquence SEQ ID NO : 58). Les conditions expérimentales optimisées en termes de concentration, solvants et temps d'incubation peuvent être déterminées par l'homme de métier.

L'invention concerne aussi un polypeptide de séquence SEQ ID NO : 2 tel qu'obtenu par le procédé précédemment défini.

Selon un autre aspect, l'invention concerne une composition pharmaceutique comprenant comme principe actif un polypeptide tel que précédemment décrit et éventuellement un ou plusieurs excipients pharmaceutiquement acceptables.

Les composés peptidiques selon l'invention peuvent être utilisés dans une composition pharmaceutique ou pour la réalisation d'un médicament. Dans ces compositions ou médicaments, le principe actif peut être incorporé dans des compositions se présentant sous des formes diverses, à savoir sous forme de solutions, en général aqueuses, ou sous forme lyophilisée, ou sous forme de gel ou d'hydrogel, ou sous forme d'émulsion ou bien toute autre forme pharmaceutiquement et physiologiquement acceptable.

Les doses à utiliser peuvent aller de 1 µg/kg à 1000 µg/kg et les voies d'administration utilisables peuvent être choisies parmi les voies intrarachidienne, intrathécale, intraventriculaire, épidurale, intraparenchymateuse, intravitréale, transphénoïdale ou topique.

D'autres voies d'administration connues de l'homme du métier peuvent également être utilisées telles que les voies sous-cutanée, intraveineuse, intraluminale, ou intranasale. Lorsque l'une ou plusieurs de ces voies d'administration sont utilisées, les doses à utiliser peuvent aller de 1 µg/kg à 50 mg/kg.

Ces médicaments ou compositions sont notamment destinés au traitement des maladies neurodégénératives et / ou à la régénération des cellules du système nerveux central (cerveau, moelle épinière) ou des nerfs périphériques après traumatismes. Leur utilisation dans la régénération des cellules du système nerveux peut se faire soit par administration directe à un patient, soit de manière extracorporelle. Ces médicaments ou compositions peuvent notamment être utilisés dans le traitement de la maladie d'Alzheimer, la sclérose en plaque, la maladie de Parkinson, ou toute autre pathologie neurodégénérative, ou les pathologies de type accidentel ou traumatique tels que lésions de la moelle épinière, les traumatismes crâniens ou les accidents vasculaires cérébraux. Ces médicaments ou compositions peuvent également être utilisés dans le traitement des lésions des nerfs auditifs, optiques, olfactifs ou de tout nerf crânien ou périphérique qu'elles soient d'origine traumatique, accidentelle ou dégénérative.

Selon un autre aspect, l'invention concerne un polypeptide tel que précédemment décrit, pour son utilisation dans le traitement des pathologies neurodégénératives ou des traumatismes dans lesquels la régénération du système nerveux central est recherchée. Le polypeptide selon la présente invention peut être utilisé pour le traitement de lésions traumatiques ou non du système nerveux périphérique.

Selon un autre mode de réalisation avantageux, l'invention concerne un polypeptide tel que précédemment décrit pour son utilisation dans le traitement de pathologies de type accidentel, traumatique ou dégénératif, notamment la maladie d'Alzheimer, la sclérose en plaque, la maladie de Parkinson, les accidents vasculaires cérébraux, la lésion médullaire, les traumatismes crâniens ou les lésions des nerfs optiques, olfactifs, auditifs ou de tout nerf crânien ou périphérique.

### DESCRIPTION DES FIGURES

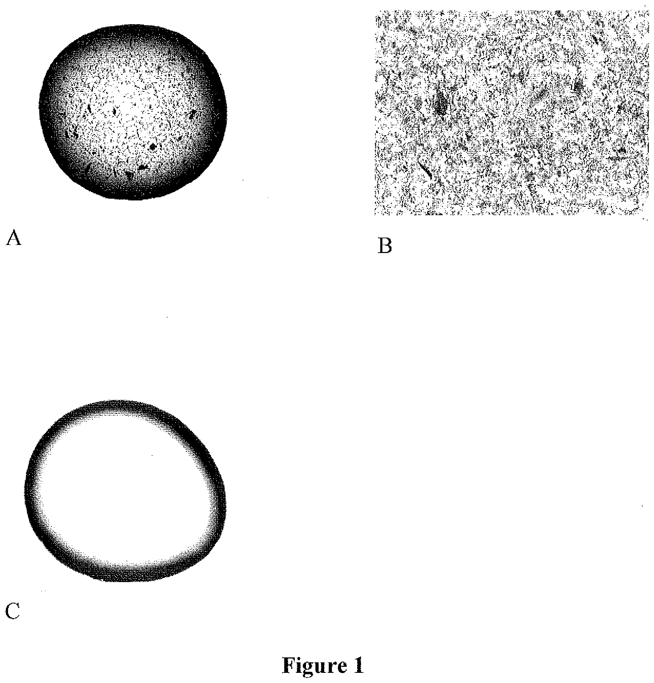

**Figure 1** **:** La Figure 1 représente l'observation au microscope optique d'une goutte d'une solution du polypeptide de séquence SEQ ID NO : 58 (A, grossissement x 25 ; B, grossissement x 100) et du polypeptide de séquence SEQ ID NO : 2 (C, grossissement x 25) à une concentration de 29,4 mg/ml dans du NaCl 0,9%.

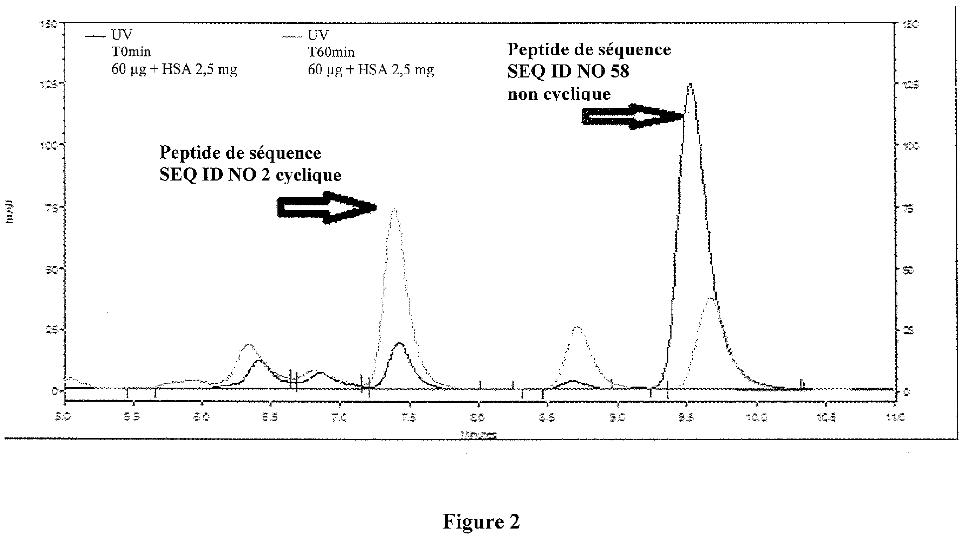

**Figure 2** **:** La Figure 2 représente les chromatogrammes superposés de la réaction de cyclisation du polypeptide de séquence SEQ ID NO : 58 en polypeptide de séquence SEQ ID NO : 2 en présence d'albumine humaine réalisés à l'initiation de la réaction (T0min) et après 60 min de réaction de cyclisation (T60 min).

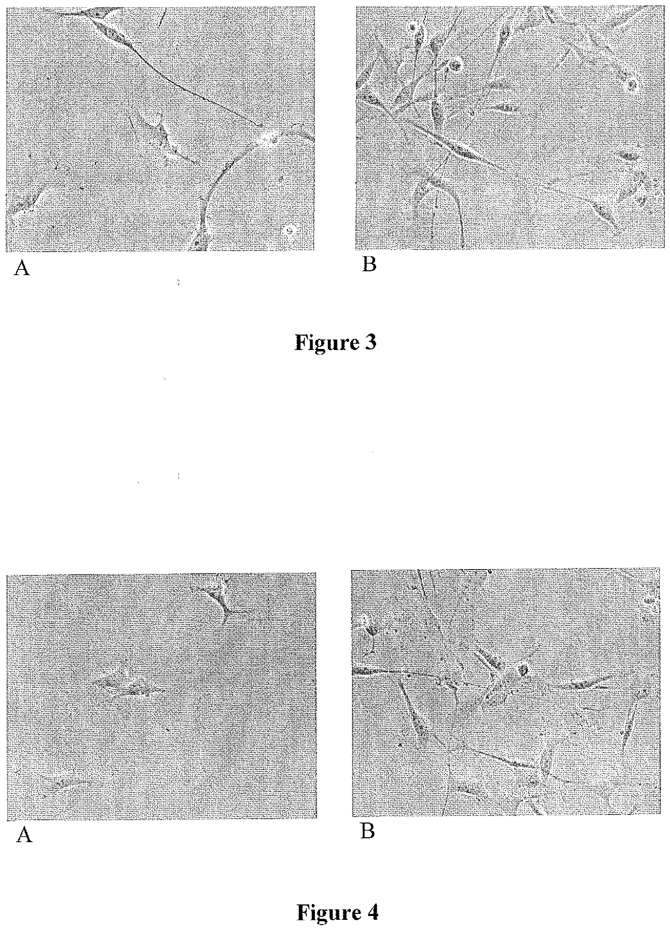

**Figure 3** : La Figure 3 représente l'observation au microscope optique à contraste de phase (grossissement x 400) de l'effet du polypeptide cyclique de séquence SEQ ID NO : 2 sur les cellules B104 après 48h. Les cellules sont cultivées pendant 2 jours dans un milieu sans sérum et en absence (A) ou présence (B) du polypeptide cyclique de séquence SEQ ID NO : 2 à 1 mg/mL.
**Figure 4** : La Figure 4 représente l'observation au microscope optique à contraste de phase (grossissement x 400) de l'effet du polypeptide cyclique de séquence SEQ ID NO : 2 sur les cellules B104 après 72h. Les cellules sont cultivées pendant 3 jours dans un milieu sans sérum et en absence (A) ou en présence (B) de polypeptide cyclique de séquence SEQ ID NO : 2 à 500 µg/mL.

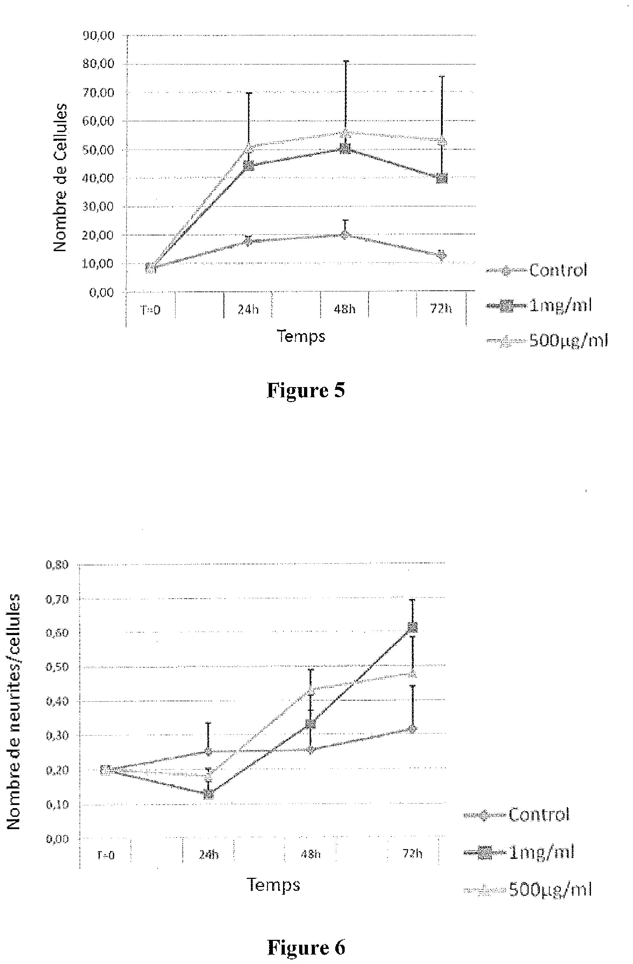

**Figure 5** : La Figure 5 représente la quantification de l'effet du polypeptide cyclique de séquence SEQ ID NO : 2 sur le nombre de cellules. Le nombre moyen de cellules B104 est analysé après 1, 2 ou 3 jours de culture. Les valeurs sont les moyennes ± SEM (n=3). Les cellules sont cultivées dans un milieu sans sérum seul (control) ou en milieu sans sérum contenant le polypeptide cyclique de séquence SEQ ID NO : 2 aux concentrations de 1 mg/mL ou 500 µg/mL.
   Le terme SEM, pour standard « error of the mean », signifie l'erreur-type de la moyenne.
**Figure 6** **:** La Figure 6 représente la quantification de l'effet du polypeptide cyclique de séquence SEQ ID NO : 2 sur le nombre de neurites. Le nombre moyen de sprouting (bourgeonnements) par cellules B104 est analysé après 1, 2 ou 3 jours de culture. Les valeurs sont les moyennes ± SEM (n=3). Les cellules sont cultivées dans un milieu sans sérum seul (control) ou en milieu sans sérum contenant du polypeptide cyclique de séquence SEQ ID NO : 2 aux concentrations de 1 mg/mL ou 500 µg/mL.

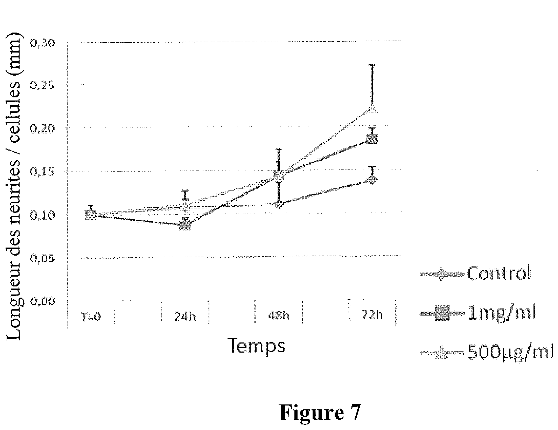

**Figure 7** **:** La Figure 7 représente la quantification de l'effet du polypeptide cyclique de séquence SEQ ID NO : 2 sur la longueur des neurites. La longueur moyenne des neurites par cellules B104 est analysée après 1, 2 ou 3 jours de culture. Les valeurs sont les moyennes ± SEM (n=3). Les cellules sont cultivées dans un milieu sans sérum seul (control) ou en milieu sans sérum contenant du polypeptide cyclique de séquence SEQ ID NO : 2 aux concentrations de 1 mg/mL ou 500 µg/mL.

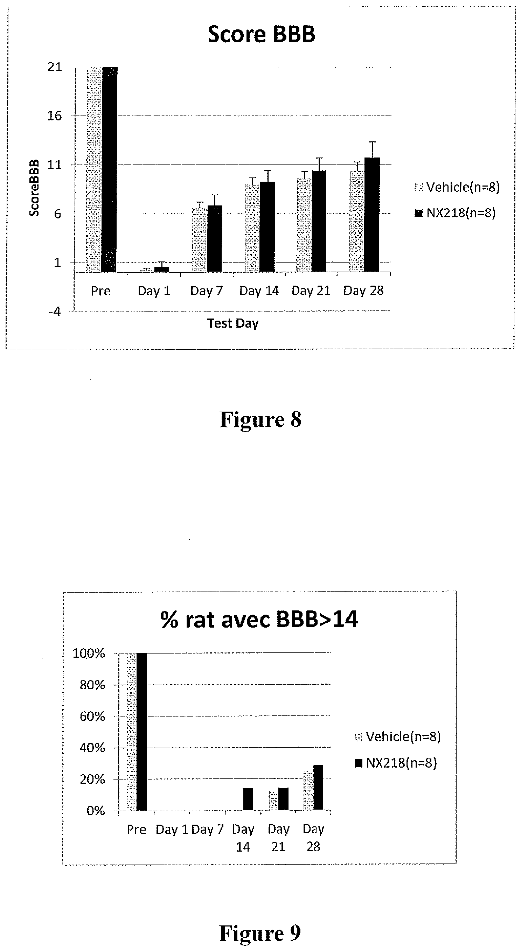

**Figure 8** : La Figure 8 représente le résultat de l'évaluation des performances locomotrices étudiées au moyen de l'échelle de Basso, Beattie, Bresnahan (BBB) (Basso DM et al. J Neurotrauma, 12, 1-21, 1995, Basso DM et al. Exp Neurol, 139, 244-256, 1996). Les valeurs sont les moyennes ± SEM. Les scores BBB s'échelonnent de 0 (pas de mouvement des pattes postérieures) à 21 (marche normale avec coordination et placement parallèle des pattes).
**Figure 9** : La Figure 9 représente le pourcentage des rats pour les lesquels le score BBB est supérieur ou égal à 14.

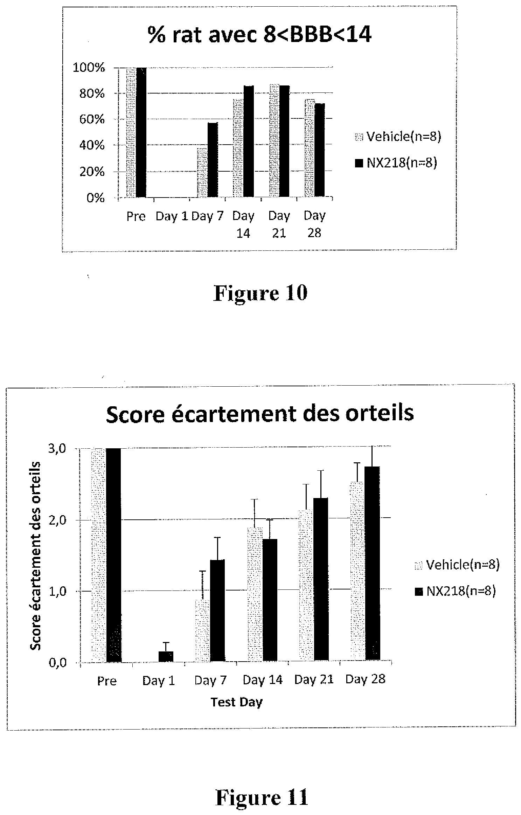

**Figure 10** : La Figure 10 représente le pourcentage des rats pour les lesquels le score BBB est compris entre supérieur ou égal à 8 et inférieur à 14.
**Figure 11** **:** La Figure 11 représente le résultat de l'évaluation des activités réflexes d'écartement des orteils. Les scores considérés sont: 0 = aucune activité réflexe, 1 = considérablement plus faible que la normale, 2 = légèrement plus faible que la normale, 3 = normale.

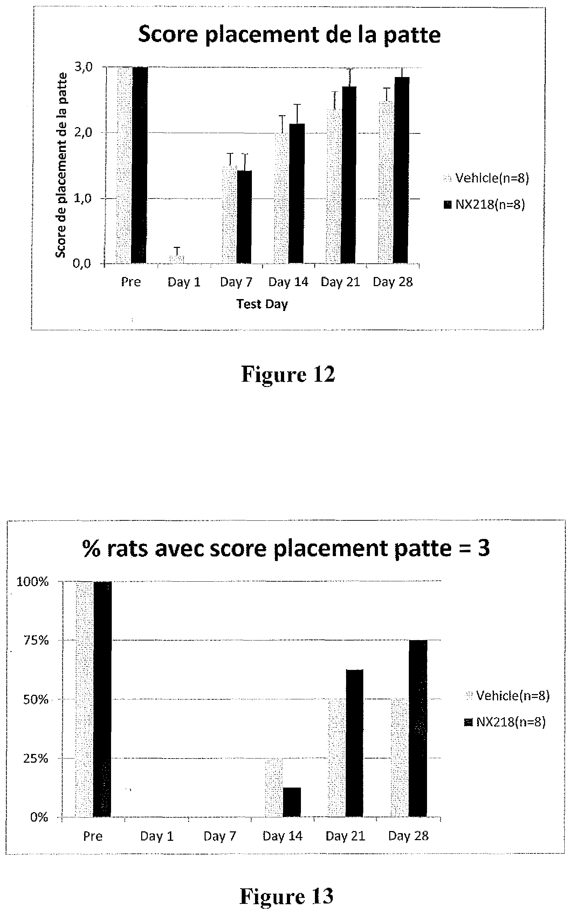

**Figure 12** **:** La Figure 12 représente le résultat de l'évaluation du placement des pattes postérieures. Les scores considérés sont: 0 = aucune activité réflexe, 1 = considérablement plus faible que la normale, 2 = légèrement plus faible que la normale, 3 = normale.
**Figure 13** : La Figure 13 représente le pourcentage des rats pour les lesquels le score du placement des pattes postérieures est égal à 3 (normal).

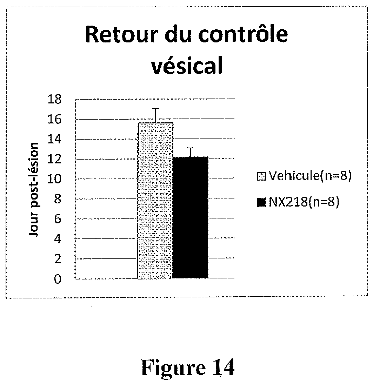

**Figure 14** **:** La Figure 14 représente la moyenne du jour auquel les rats ont recouvré le contrôle vésical. Les valeurs sont les moyennes ± SEM.

### EXEMPLES :

### Exemple 1 : Méthodes de Synthèse du polypeptide de séquence W-S-G-W-S-S-C-S-R-S-C-G dans laquelle les 2 cystéines sont liées par un pont disulfure (SEQ ID NO : 2) :

### 1) Oxydation du polypeptide de séquence W-S-G-W-S-S-C-S-R-S-C-G (SEQ ID NO : 58) en présence d'albumine humaine

Pour réaliser cette synthèse, nous avons mis en présence le polypeptide de séquence W-S-G-W-S-S-C-S-R-S-C-G (SEQ ID NO : 58) avec différentes quantités d'albumine de telle sorte que les ratios polypeptide de séquence SEQ ID NO : 58:albumine humaine (HSA) soient de l'ordre de 1:100, de 1:10 et de 1:1.

En partant du polypeptide de séquence W-S-G-W-S-S-C-S-R-S-C-G (SEQ ID NO : 58) mis en présence d'un ratio 1:1 de HSA et incubé pendant 1 à 3 heures à température ambiante sous agitation a l'air, nous avons observé par HPLC la formation d'un pic correspondant au polypeptide de séquence W-S-G-W-S-S-C-S-R-S-C-G dans laquelle les 2 cystéines sont liées par un pont disulfure (SEQ ID NO : 2). Apres élimination de l'albumine par précipitation, le produit est ensuite purifié et analysé par HPLC. L'utilisation de ratio différent d'albumine et de polypeptide correspondant à la séquence SEQ ID NO : 58 permet d'influencer la vitesse de cyclisation et le rendement final de la cyclisation tout en sachant qu'une quantité plus faible d'albumine est plus facile à éliminer.

### 2) Autre méthode de préparation du polypeptide correspondant à la séquence SEQ ID NO : 2 évitant la séparation et purification préalable du polypeptide linéaire (polypeptide de séquence SEQ ID NO : 58)

Le polypeptide de séquence W-S-G-W-S-S-C-S-R-S-C-G (SEQ ID NO : 58) est synthétisé sur résine d'une manière similaire à la méthode classique indiquée dans la demande de brevet international WO 1999/03890 mais sans le cliver ou le détacher de la résine. Cela permet d'éviter deux étapes couteuses en temps et argent et qui consistent à isoler puis à purifier le polypeptide avant de le remettre en solution pour l'oxyder en produit désiré correspondant à la séquence SEQ ID NO : 2. L'oxydation en présence d'albumine se fait directement avec le polypeptide de séquence W-S-G-W-S-S-C-S-R-S-C-G (SEQ ID NO : 58) lié à la résine. La purification est similaire à celle décrite dans l'exemple ci-dessus mais l'étape de séparation de l'albumine est plus facile car elle se fait par simple lavage, le polypeptide étant encore attaché à la résine. Une fois que l'albumine a été éliminée, alors le polypeptide peut être détaché de la résine par des méthodes classiques.

### Exemple 2 : Effet de l'albumine sur la cyclisation du polypeptide de séquence W-S-G-W-S-S-C-S-R-S-C-G (SEQ ID NO : 58) c'est à dire sa transformation en polypeptide de séquence W-S-G-W-S-S-C-S-R-S-C-G dans laquelle les 2 cystéines sont liées par un pont disulfure (SEQ ID NO : 2)

Nous avons étudié le comportement du polypeptide de séquence W-S-G-W-S-S-C-S-R-S-C-G (SEQ ID NO : 58) en absence et en présence d'albumine humaine (HSA) et la formation résultante du polypeptide de séquence W-S-G-W-S-S-C-S-R-S-C-G dans laquelle les 2 cystéines sont liées par un pont disulfure (SEQ ID NO : 2) .

Le procédé est le suivant :
Une solution contenant du polypeptide de séquence W-S-G-W-S-S-C-S-R-S-C-G (SEQ ID NO : 58) à 50 µg/mL a été préparée dans du PBS et glucose à 5% en présence ou absence de HSA à 2.5 mg/mL.

Le ratio polypeptide de séquence W-S-G-W-S-S-C-S-R-S-C-G (SEQ ID NO : 58) : HSA est de l'ordre de 1:1 et donc équimolaire.

Les solutions ont été mélangées et le mélange injecté directement par HPLC en phase inverse suivant une méthode utilisant une colonne Phenomenex Jupiter de 300 Å et des solvants d'élution constitués d'eau HPLC avec 0,1% d'acide trifluoroacétique et acétonitrile avec 0.1% d'acide trifluoroacétique permettant de séparer les pics de HSA, de polypeptide de séquence W-S-G-W-S-S-C-S-R-S-C-G (SEQ ID NO : 58) et de polypeptide de séquence W-S-G-W-S-S-C-S-R-S-C-G dans lequel les deux cystéines forment un pont disulfure (SEQ ID NO : 2).

Cette opération a été répétée à T=0 min et à T=60 min.

Les profils chromatographiques ont permis d'établir les observations suivantes :
- En 60 minutes, **en absence** d'albumine (HSA) et donc par oxydation à l'air, le pic de polypeptide de séquence W-S-G-W-S-S-C-S-R-S-C-G dans lequel les deux cystéines forment un pont disulfure (SEQ ID NO : 2) augmente de 9.1% par rapport au pic de départ du polypeptide de séquence SEQ ID NO : 58.
- Dans des conditions identiques, mais **en présence** d'albumine, à T= 60 min, le pic de polypeptide de séquence W-S-G-W-S-S-C-S-R-S-C-G dans lequel les deux cystéines forment un pont disulfure (SEQ ID NO : 2) augmente de 38% par rapport au pic de départ du polypeptide de séquence SEQ ID NO : 58.

Ce résultat montre un effet accélérateur de l'albumine sur la cyclisation du polypeptide de séquence SEQ ID NO : 58 qui conduit à la formation du polypeptide de séquence W-S-G-W-S-S-C-S-R-S-C-G dans lequel les deux cystéines forment un pont disulfure (SEQ ID NO : 2).
- En **absence d'albumine,** en 60 minutes, 25% du pic du polypeptide de séquence SEQ ID NO : 58 se transforme en polypeptide de séquence W-S-G-W-S-S-C-S-R-S-C-G dans lequel les deux cystéines forment un pont disulfure (SEQ ID NO : 2)
- En **présence d'albumine** et dans les mêmes conditions, en 60 minutes, 54% du polypeptide de séquence SEQ ID NO : 58 se transforme en polypeptide de séquence W-S-G-W-S-S-C-S-R-S-C-G dans lequel les deux cystéines forment un pont disulfure (SEQ ID NO : 2)

Ce résultat montre qu'en présence d'albumine, une plus grande quantité de produit correspondant à la SEQ ID NO : 2 se forme en comparaison avec l'oxydation par l'air, ce qui correspond à une spécificité et une pureté supérieures en faveur de l'albumine.

Discussion :
Les deux expériences décrites dans cet exemple montre le rôle important de l'albumine pour produire du polypeptide de séquence W-S-G-W-S-S-C-S-R-S-C-G dans lequel les deux cystéines forment un pont disulfure (SEQ ID NO : 2) à partir du polypeptide de séquence W-S-G-W-S-S-C-S-R-S-C-G (SEQ ID NO : 58) en comparaison d'une oxydation à l'air. La réaction est en effet au minimum 2 fois plus rapide, préférablement 5 fois plus rapide, et plus préférablement 10 fois plus rapide en fonction de ratios peptide/albumine à l'air. Dans l'exemple indiqué ci-dessus, on observe la formation de 9.1% sans albumine contre 38 % avec albumine, donc la réaction est ici environ 4 fois plus rapide et elle est aussi effectuée avec une meilleure spécificité (25% sans albumine contre 54% avec albumine) ce qui correspond à une plus grande pureté. En effet, en 60 minutes, 25% de transformation du polypeptide de séquence W-S-G-W-S-S-C-S-R-S-C-G (SEQ ID NO: 58) en absence d'albumine correspond à la formation possible de 75% d'impuretés et en présence d'albumine, 54% de transformation du polypeptide de séquence W-S-G-W-S-S-C-S-R-S-C-G (SEQ ID NO : 58) correspond à la formation de seulement 46% d'impuretés.

### Exemple 3 : Effet des ratios du polypeptide de séquence W-S-G-W-S-S-C-S-R-S-C-G (SEQ ID NO: 58) par rapport à l'albumine sur l'efficacité de la cyclisation du polypeptide de séquence W-S-G-W-S-S-C-S-R-S-C-G (SEQ ID NO : 58) en polypeptide de séquence W-S-G-W-S-S-C-S-R-S-C-G dans laquelle les 2 cystéines sont liées par un pont disulfure (SEQ ID NO : 2)

L'exemple 2 a montré comment un ratio équimolaire (ratio molaire identique) d'albumine et de polypeptide permettait d'obtenir une quantité supérieure de polypeptide correspondant à la séquence SEQ ID NO : 2 à partir du polypeptide de séquence SEQ ID NO : 58 et avec une pureté supérieure. L'exemple ci-dessous décrit comment des ratios différents d'albumine et de polypeptide influencent la vitesse de formation du pont disulfure et la pureté du polypeptide de séquence SEQ ID NO : 2.

Trois quantités de polypeptide de séquence W-S-G-W-S-S-C-S-R-S-C-G (SEQ ID NO : 58) sont incubées environ 1 heure à 37°C dans un même volume de liquide céphalorachidien de rat dans lequel se trouve une quantité physiologique constante d'albumine et la vitesse de formation du polypeptide cyclique de séquence W-S-G-W-S-S-C-S-R-S-C-G dans lequel les deux cystéines forment un pont disulfure (SEQ ID NO : 2) est observée par HPLC. L'étude cherche à démontrer l'influence du ratio polypeptide:albumine sur la vitesse de cyclisation du polypeptide linéaire de séquence SEQ ID NO : 58 mesurée par le Tmax.

Le Tmax, correspondant au temps où la quantité la plus grande de polypeptide cyclique de séquence W-S-G-W-S-S-C-S-R-S-C-G dans lequel les deux cystéines forment un pont disulfure (SEQ ID NO : 2) est formée, est rapporté dans la Table 1.

Les ratios correspondant aux différentes doses de polypeptide de séquence SEQ ID NO : 58 ajoutées sont indiqués dans la Table 1.

**Table 1 : Effet des ratios polypeptide de séquence SEQ ID NO : 58 : albumine sur le Tmax de la réaction de cyclisation du polypeptide de séquence SEQ ID NO : 58 en polypeptide de séquence SEQ ID NO : 2**

| | **Cyclisation en présence d'albumine** | | |
|---|---|---|---|
| **Masse de polypeptide de séquence SEQ ID NO : 58 ajoutée (mg)** | 6,00 | 60 | 600 |
| **Ratio polypeptide:albumine** | 2:1 | 20:1 | 200:1 |
| **Tmax (min)** | 10 | 30 | 120 |

Conclusion : Un effet de catalyseur pour l'albumine est observé, tel qu'exemplifié par les ratios présentés dans la table 1 qui démontrent l'implication de l'albumine dans l'accélération de la cyclisation. En effet une très légère différence dans la quantité d'albumine mise en présence du polypeptide (telle que représentée par les ratios 20:1 et 200:1) permet d'accélérer la cyclisation et par conséquent réduire son Tmax de 120 à 30 minutes.

Pour conclure, il y a une corrélation entre le ratio de polypeptide de séquence W-S-G-W-S-S-C-S-R-S-C-G (SEQ ID NO : 58) par rapport à l'albumine et la cinétique de cyclisation.

En effet, plus petit est le ratio polypeptide : albumine, et plus rapide est la réaction. En d'autres termes, plus le ratio entre le polypeptide et l'albumine se rapproche de 1:1 et plus la réaction est rapide. Finalement, une petite quantité d'albumine est suffisante pour accélérer la réaction de cyclisation et la formation de disulfure (effet catalyseur).

Alternativement, la cyclisation et production de disulfure en présence d'albumine peut se faire en absence complète d'air ou en présence d'une quantité très faible d'air, et donc d'oxygène, ce qui peut permettre d'éviter la formation de produits contaminants venant de réactions d'oxydation non contrôlées.

### Exemple 4: Etude de la solubilité du polypeptide cyclique de séquence W-S-G-W-S-S-C-S-R-S-C-G dans lequel les deux cystéines forment un pont disulfure (SEQ ID NO : 2) en solution aqueuse de NaCl

Deux pesées de 0,5 mg sont réalisées dans un microtube d' 1.5 mL pour le polypeptide
W-S-G-W-S-S-C-S-R-S-C-G (SEQ ID NO : 58) et le polypeptide :

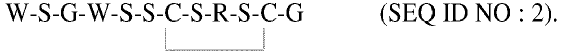
W-S-G-W-S-S-C-S-R-S-C-G          (SEQ ID NO : 2).

Dans chaque tube sont ajoutés 17 microlitres d'une solution de NaCl 0,9% précédemment préparée afin de réaliser une solution peptidique à 29,4 mg/mL. Les tubes sont alors agités au vortex pendant quelques secondes et une goutte de chaque solution de polypeptide obtenue est placée sur une lame en verre pour observation au microscope optique. Dans ces conditions, seul le polypeptide de séquence SEQ ID NO : 2 apparait soluble sans aucune particule ou agrégat visible (Figure 1). Le polypeptide de séquence SEQ ID NO : 58 présente un nombre important de particules.

La limite de solubilité du polypeptide de séquence SEQ ID NO : 58 dans une solution aqueuse de NaCl à 0,9% est déterminée à environ 2 mg/mL au microscope et par examen visuel.

La solubilité du polypeptide de séquence SEQ ID NO : 2 dans NaCl 0,9% est estimée être supérieure à 29 mg/mL par examen visuel.

La limite de solubilité du polypeptide de séquence SEQ ID NO : 2 dans une solution de NaCl 0,9% est donc au moins 15 fois supérieure à celle du polypeptide de séquence SEQ ID NO : 58 dans la même solution.

### Exemple 5 : Meilleure stabilité du polypeptide W-S-G-W-S-S-C-S-R-S-C-G dans laquelle les 2 cystéines sont liées par un pont disulfure (SEQ ID NO : 2) contre le W-S-G-W-S-S-C-S-R-S-C-G (SEQ ID NO : 58) en présence d'enzymes

Une digestion enzymatique a été réalisée mettant en oeuvre de la néprylysine incubée avec la forme purifiée du polypeptide d'intérêt soit sous sa forme linéaire (SEQ ID NO : 58) soit sous sa forme cyclique (SEQ ID NO : 2). Les paramètres temps (t=0 et t=30 minutes) ont été explorés avec une enzyme diluée au dixième dans l'eau. Un échantillon dit « témoin » sans enzyme et mené en parallèle a été réalisé pour établir une référence analytique lors de chaque paramètre exploré.

Les polypeptides sont dilués dans de l'eau désoxygénée afin de maintenir la stabilité des deux formes au cours du temps. Les digestions ont été analysées par la technique de spectrométrie de masse LC/MS/MS.

Les résultats (aires sous la courbe et pourcentage de digestion) sont présentés dans la Table 2. Le pourcentage de digestion correspond, pour un polypeptide donné, au pourcentage de l'aire sous la courbe en présence d'enzyme par rapport à l'aire sous la courbe du témoin pour ce même polypeptide.

**Table 2 : Aires sous la courbe mesurée après analyse de la digestion des polypeptides par la néprylisine par LC/MS/MS et pourcentage de digestion pour le polypeptide de séquence SEQ ID NO : 58 et le polypeptide de séquence SEQ ID NO : 2.**

| | | **Polypeptide de séquence SEQ ID NO : 58** | **Polypeptide de séquence SEQ ID NO : 2** |
|---|---|---|---|
| T=0 min | Aire sous la courbe du témoin (absence d'enzyme) | 788400 | 3008000 |
| | Aire sous la courbe en présence d'enzyme | 797200 | 3220000 |
| | Pourcentage de digestion | aucune | aucune |
| T=30 min | Aire sous la courbe du témoin (absence d'enzyme) | 802800 | 3722000 |
| | Aire sous la courbe en présence d'enzyme | 576500 | 3401000 |
| | Pourcentage de digestion | 28% | 8,5% |

Conclusion : A t=0, les deux polypeptides semblent stables en présence de néprilysine. A t=30 min, le peptide cyclique (SEQ ID NO : 2) est plus stable que le polypeptide linéaire (SEQ ID NO : 58) (28% de digestion contre 8,5%).

### Exemple 6 : Séparation des deux pics correspondant aux polypeptides de séquences SEQ ID NO : 2 et SEQ ID NO : 58 montrant la différence de conformation

Une synthèse de polypeptide de séquence SEQ ID NO : 2 à partir de polypeptide de SEQ ID NO : 58 en présence d'albumine humaine est effectuée. Deux analyses par HPLC des composés présents sont faites au cours de la réaction de synthèse: la première à T= 0 minute, et la deuxième à T = 60 minutes. Les chromatogrammes obtenus sont présentés superposés en Figure 2.

Les conditions analytiques pratiquées pour la séparation des composés sont les suivantes:

| | |
|---|---|
| • Colonne: | XBridge^{®} C18, 2.1 x 100 mm, 3.5 µm |
| • Phase mobile: | A: acétonitrile avec 0.1% de TFA (Acide trifluoro acétique) |
| | B: eau distillé contenant 0.1% de TFA |
| • Vitesse d'élution: | 0.500 mL/min |

- Gradient:

| Time (min) | A% | B% |
|---|---|---|
| 0 | 10 | 90 |
| 11 | 25 | 75 |
| 21 | 10 | 90 |

La Figure 2 montrent que les pics correspondant aux polypeptides de séquences SEQ ID NO : 2 (cyclique) et SEQ ID NO : 58 (linéaire, non cyclique) peuvent être facilement séparés.

### Example 7 : Pharmacologie in vitro

Des cellules de neuroblastome de la lignée B104 sont mises en culture en flasque de 75 cm² à 37°C sous 5% de CO₂ dans le milieu Dulbecco's Modified Eagle's Medium (DMEM) supplémenté avec de la glutamine à 2 mM, de la pénicilline G à 50 U/mL, du sulfate de streptomycine à 50 µg/mL et 10% de sérum foetal bovin (FBS). Les cellules sont ensemencées dans des plaques de 48 puits recouvertes d'une matrice de poly-D-lysine à 10 µg/mL pour obtenir une densité cellulaire finale de 5 000 cellules/puits. Quatre heures après l'ensemencement, le milieu de culture est remplacé par du milieu sans FBS à raison de 200 µL/puits contenant ou non du polypeptide cyclique de séquence SEQ ID NO : 2 à une concentration de 1000, 500, 375 ou 150 µg/mL. Le milieu de culture n'est plus changé pendant toute la durée de l'expérimentation. L'absence de sérum va alors induire une initiation de la différentiation des B104 avec apparitions d'extensions neuritiques plus ou moins importantes.

Pour quantifier les effets du polypeptide cyclique de séquence SEQ ID NO : 2 sur les cultures, les cellules sont observées après 1, 2 et 3 jours de culture dans des champs sélectionnés au hasard sous microscope à contraste de phase en utilisant un objectif quadrillé (1×1 cm, divisé en carrés 10x10) et à grossissement x 400. Chaque puits est compté en triplicat pour chaque expérimentation et 3 champs par puits sont examinés. Les paramètres analysés sont le nombre de cellules, le nombre de neurites (extension cellulaire au moins aussi grande que le diamètre du corps de la cellule) par cellules et la longueur moyenne des 10 plus longs neurites.
- Dans ces conditions, on observe que : Le peptide cyclique de séquence SEQ ID NO : 2 a un effet sur le **nombre** de cellules B104:
   En condition standard, c'est-à-dire en absence du polypeptide de séquence SEQ ID NO : 2, la croissance des cellules B104 augmente pendant 48h avant que le nombre de cellules ne commence à décroitre dû à l'absence de sérum et d'un appauvrissement du milieu de culture. Cependant, en présence du polypeptide cyclique de séquence SEQ ID NO : 2, le nombre de cellules est augmenté dès 24h avec 5 fois plus de cellules comparé à la condition contrôle.
   Des exemples de croissance de cellules B104 sont présentés en Figures 2, 3, 4 et 5.
- Le polypeptide cyclique de séquence SEQ ID NO : 2 a un effet sur la **morphologie** des cellules B104:
   Alors que les cellules B104 ont une morphologie de type fibroblastique lorsqu'elles sont cultivées en présence de sérum, elles s'engagent dans une différenciation de type neuronal lorsqu'elles sont cultivées en absence de sérum. A 24h, en culture sans sérum et quelle que soit la présence ou l'absence de facteurs de différenciation, les cellules B104 montrent des extensions mono ou bipolaires ainsi que des bourgeonnements (sprouting, petites extensions moins grandes que le diamètre du corps cellulaire) et des neurites (extensions cellulaires au moins aussi grandes que le diamètre du corps de la cellule), morphologie typique de ces conditions (Schubert D et al., Nature. 249(454), 224-227 1974).

Le polypeptide cyclique de séquence SEQ ID NO : 2, quelle que soit sa concentration, n'a aucun effet sur le nombre de sprouting. Cependant, la présence du polypeptide cyclique de séquence SEQ ID NO : 2 augmente le nombre de neurites par cellules (Figure 6) et leurs longueurs (Figure 7).

Après 3 jours de culture, les B104 traitées ont développé des extensions neuritiques importantes et qui ont crû au fur et à mesure du temps de culture. Cet effet sur la longueur des neurites apparait dose-dépendant.

### Exemple 8 : Pharmacologie In vivo - Evaluation sur un modèle de lésion médullaire

Le modèle de contusion est reconnu et utilisé pour mimer la lésion médullaire observée chez l'homme (Young W., Prog Brain Res., 137, 231-55, 2002).

Ce modèle est utilisé pour évaluer l'efficacité de principes actifs sur le recouvrement fonctionnel. Les expérimentations sont réalisées sur des rats adultes femelles Sprague-Dawley d'environ 250 g. La procédure chirurgicale se fait sous anesthésie générale avec 5% isoflurane (dans 70 % N₂O et 30 % O₂; débit 300 ml/min).

Après laminectomie au niveau des vertèbres thoraciques T9 et T10, la lésion médullaire est obtenue par utilisation d'un dispositif à commande électromagnétique (système PinPoint, Hatteras Inc., USA) permettant d'obtenir une contusion calibrée (profondeur 2 mm, diamètre de l'impacteur 2 mm, propulsé à la vitesse de 1,5 m/s pour 85 ms) (d'après Bilgen M. Neurorehab and Neuralre. 19(3), 2005).

Dans les quinze minutes suivant le traumatisme, 3 µL du polypeptide cyclique de séquence SEQ ID NO : 2 à une concentration de 7 µg/mL ou le véhicule est injecté au moyen d'une seringue Hamilton (10 pi, modèle 1701) et d'un système de micro-perfusion (Harvard Apparatus) permettant l'injection dans l'espace intra-rachidien. Après injection, l'aiguille est laissée en place 5 min. Dès que l'aiguille est retirée un adhésif cellulaire est appliqué sur le point d'injection pour colmater la dure mère et éviter toute fuite de fluide biologique. Après suture cutanée, les rats sont hébergés individuellement dans des cages standard à la température de 22°C ± 1°C sous éclairage contrôlé (12h/jour), eau et alimentation *ad libitum.* Une seconde injection est réalisée 2 jours plus tard. Les tests comportementaux sont réalisés sur chaque animal observé individuellement, en aveugle.

Les performances locomotrices sont étudiées au moyen de l'échelle de Basso, Beattie, Bresnahan (BBB) (Basso DM et al. J Neurotrauma, 12, 1-21, 1995, Basso DM et al. Exp Neurol, 139, 244-256, 1996).

Chaque animal est évalué avant le traumatisme puis une fois par semaine. Parallèlement, chaque animal est pesé avant le traumatisme puis une fois par semaine.

Dans l'échelle d'évaluation de la locomotion: score BBB, les données statistiques sont exprimées par leurs moyennes ± SEM.

Les scores BBB s'échelonnent de 0 (pas de mouvement des pattes postérieures) à 21 (marche normale avec coordination et placement parallèle des pattes). Les scores de 0 à 7 indiquent le retour de mouvements isolés des trois articulations des pattes postérieures (cheville, genoux, hanche), de 8 à 13 indiquent le retour du placement des pattes postérieures et de la coordination avec les pattes antérieures, de 14 à 21 indiquent le retour de l'écartement des orteils, de la position des pattes et de la queue, et de la stabilité du tronc.

L'évaluation des scores BBB est réalisée avant lésion, puis aux jours 1, 7, 14, 21 et 28 posttraitement par deux observateurs indépendants (Figures 8, 9, 10).

Les activités réflexes d'écartement des orteils (Figure 11) et du placement des pattes postérieures (Figures 12 et 13) sont analysées aux jours 1, 7, 14, 21 et 28 post-traitement. Les scores considérés sont: 0 = aucune activité réflexe, 1 = considérablement plus faible que la normale, 2 = légèrement plus faible que la normale, 3 = normale.

Le jour du retour du contrôle vésical est également un paramètre mesuré (Figure 14).

## Revendications

1. Polypeptide consistant en la séquence d'acides aminés suivante :
W-S-G-W-S-S-C-S-R-S-C-G (SEQ ID NO : 2) dans laquelle les deux cystéines forment un pont disulfure.

2. Polypeptide selon la revendication 1, ledit polypeptide étant pratiquement dépourvu des formes réduite ou dimérique ou oligomérique dudit polypeptide ainsi que des dérivés dudit polypeptide dans lesquels les groupements thiols sont sous forme de sulfoxide ou de sulfone.

3. Polypeptide selon la revendication 1 ou 2, ledit polypeptide présentant une pureté supérieure à 80%, de préférence 85%, plus préférentiellement 90%, plus préférentiellement encore égale ou supérieure à 95%.

4. Procédé d'obtention du polypeptide de séquence d'acides aminés SEQ ID NO : 2:
W-S-G-W-S-S-C-S-R-S-C-G (SEQ ID NO : 2)
dans laquelle les deux cystéines forment un pont disulfure,
le procédé comprenant une étape de formation d'un pont disulfure en présence d'albumine dans le polypeptide de séquence SEQ ID NO : 58 :
W-S-G-W-S-S-C-S-R-S-C-G (SEQ ID NO : 58).

5. Procédé d'obtention du polypeptide de séquence SEQ ID NO : 2 selon la revendication 4, **caractérisé en ce que** l'albumine et le polypeptide de séquence SEQ ID NO : 58 sont présents dans un ratio de 1:1 à 1:100, de préférence de 1:1 à 1:10 et plus préférentiellement de 1:1.

6. Procédé d'obtention du polypeptide de séquence SEQ ID NO : 2 selon l'une des revendications 4 ou 5, **caractérisé en ce que** l'étape de formation d'un pont disulfure en présence d'albumine dans le polypeptide de séquence SEQ ID NO : 58 est réalisée à l'air ambiant.

7. Procédé d'obtention du polypeptide de séquence SEQ ID NO : 2 selon l'une des revendications 4, 5 ou 6, **caractérisé en ce que** l'étape de formation d'un pont disulfure en présence d'albumine dans le polypeptide de séquence SEQ ID NO : 58 est réalisée sans détacher le polypeptide de séquence SEQ ID NO : 58 de la résine utilisée pour la synthèse peptidique de ce polypeptide et **en ce que** le polypeptide de séquence SEQ ID NO : 2 est ensuite obtenu en séparant le polypeptide de la résine après l'étape de formation du pont disulfure.

8. Composition pharmaceutique comprenant comme principe actif un polypeptide tel que décrit à l'une des revendications 1 à 3 et un ou plusieurs excipients pharmaceutiquement acceptables.

9. Polypeptide selon l'une des revendications 1 à 3, pour son utilisation dans le traitement des pathologies neurodégénératives ou des traumatismes dans lesquels la régénération du système nerveux central est recherchée.

10. Polypeptide selon l'une des revendications 1 à 3, pour son utilisation dans le traitement de pathologies de type accidentel, traumatique ou dégénératif, notamment la maladie d'Alzheimer, la sclérose en plaque, la maladie de Parkinson, la lésion, les traumatismes crâniens ou les lésions des nerfs optiques, olfactifs, auditifs ou de tout nerf crânien ou périphérique.

## Patentansprüche

1. Polypeptid, das aus der folgenden Aminosäurensequenz besteht:
W-S-G-W-S-S-C-S-R-S-C-G (SEQ ID NO : 2) wobei die zwei Cysteine eine Disulfidbrücke bilden.

2. Polypeptid nach Anspruch 1, wobei das Polypeptid praktisch frei von der reduzierten oder Dimer- oder Oligomerform des Polypeptids sowie den Derivaten des Polypeptids ist, in denen die Thiolgruppen in Form von Sulfoxid oder von Sulfon vorliegen.

3. Polypeptid nach Anspruch 1 oder 2, wobei das Polypeptid eine Reinheit von über 80 %, vorzugsweise 85 %, vorzugsweiser 90 %, noch vorzugsweiser von gleich oder über 95 % aufweist.

4. Verfahren zur Herstellung des Polypeptids der Aminosäurensequenz (SEQ ID NO : 2):
W-S-G-W-S-S-C-S-R-S-C-G (SEQ ID NO : 2)
wobei die zwei Cysteine eine Disulfidbrücke bilden,
wobei das Verfahren einen Schritt des Bildens einer Disulfidbrücke bei Anwesenheit von Albumin im Polypeptid der Sequenz SEQ ID NO : 58 umfasst:
W-S-G-W-S-S-C-S-R-S-C-G (SEQ ID NO : 58).

5. Verfahren zur Herstellung des Polypeptids der Sequenz SEQ ID NO : 2 nach Anspruch 4, **dadurch gekennzeichnet, dass** das Albumin und das Polypeptid der Sequenz SEQ ID NO : 58 in einem Verhältnis von 1:1 zu 1:100, vorzugsweise von 1:1 zu 1:10 und vorzugsweiser von 1:1 vorhanden sind.

6. Verfahren zur Herstellung des Polypeptids der Sequenz SEQ ID NO : 2 nach einem der Ansprüche 4 oder 5, **dadurch gekennzeichnet, dass** der Schritt des Bildens einer Disulfidbrücke bei Anwesenheit von Albumin im Polypeptid der SEQ ID NO : 58 in der Umgebungsluft durchgeführt wird.

7. Verfahren zur Herstellung des Polypeptids der Sequenz SEQ ID NO : 2 nach einem der Ansprüche 4, 5 oder 6, **dadurch gekennzeichnet, dass** der Schritt des Bildens einer Disulfidbrücke bei Anwesenheit von Albumin im Polypeptid der Sequenz SEQ ID NO : 58 durchgeführt wird, ohne das Polypeptid der Sequenz SEQ ID NO : 58 von dem Harz zu lösen, das für die Peptidsynthese dieses Polypeptids verwendet wird, und dass das Polypeptid der Sequenz SEQ ID NO : 2 danach durch Trennen des Polypeptids vom Harz nach dem Schritt des Bildens der Disulfidbrücke erhalten wird.

8. Pharmazeutische Zusammensetzung, die als Wirkstoff ein Polypeptid nach einem der Ansprüche 1 bis 3 und gegebenenfalls einen oder mehrere pharmazeutisch akzeptable Hilfsstoffe umfasst.

9. Polypeptid nach einem der Ansprüche 1 bis 3 für dessen Verwendung bei der Behandlung neurodegenerativer Erkrankungen oder von Traumen, bei denen die Regenerierung des Zentralnervensystems angestrebt wird.

10. Polypeptid nach einem der Ansprüche 1 bis 3 für dessen Verwendung bei der Behandlung von Erkrankungen von Typ Unfall, Trauma oder Degeneration, insbesondere der Alzheimer-Krankheit, der Multiplen Sklerose, der Parkinson-Krankheit, der Läsion, der Schädeltraumen oder der Läsionen des Seh-, Riech-, Hör- oder jedes anderen Schädel- oder peripheren Nervs.

## Claims

1. Polypeptide consisting in the following amino acid sequence:
W-S-G-W-S-S-C-S-R-S-C-G (SEQ ID NO: 2) wherein the two cysteines form a disulfide bridge.

2. Polypeptide according to claim 1, said polypeptide being substantially free of reduced or dimeric or oligomeric forms of the polypeptide as well as derivatives of the polypeptide in which the thiol groups are in the form of sulfoxide or sulfone.

3. Polypeptide according to claim 1 or 2, wherein said polypeptide has a purity greater than 80%, preferably 85%, more preferably 90%, even more preferably equal to or greater than 95%.

4. Method for obtaining the polypeptide of amino acid sequence SEQ ID NO: 2:
W-S-G-W-S-S-C-S-R-S-C-G (SEQ ID NO: 2)
in which the two cysteines form a disulfide bridge,
said method comprising a step of forming a disulfide bridge in the presence of albumin in the polypeptide of sequence SEQ ID NO: 58:
W-S-G-W-S-S-C-S-R-S-C-G (SEQ ID NO: 58).

5. Method for obtaining the polypeptide of sequence SEQ ID NO: 2 according to claim 4, **characterized in that** albumin and the polypeptide of sequence SEQ ID NO: 58 are present in a ratio of 1 : 1 to 1 : 100, preferably 1 : 1 to 1 : 10, and more preferably 1:1.

6. Method for obtaining the polypeptide of sequence SEQ ID NO: 2 according to one of claims 4 or 5, **characterized in that** the step of forming a disulfide bridge in the presence of albumin in the polypeptide of sequence SEQ ID NO: 58 is carried out in ambient air.

7. Method for obtaining the polypeptide of sequence SEQ ID NO: 2 according to one of claims 4, 5 or 6, **characterized in that** the step of forming a disulfide bridge in the presence of albumin in the polypeptide of sequence SEQ ID NO: 58 is carried out without detaching the polypeptide sequence SEQ ID NO: 58 from the resin used for the peptide synthesis of this polypeptide, and **in that** the polypeptide of sequence SEQ ID NO: 2 is then obtained by separating the polypeptide from the resin after the disulfide bond formation step.

8. Pharmaceutical composition comprising as active ingredient a polypeptide as described in one of claims 1 to 3 and one or more pharmaceutically-acceptable excipients.

9. Polypeptide according to one of claims 1 to 3, for its use in the treatment of neurodegenerative pathologies or trauma in which the regeneration of the central nervous system is sought.

10. Polypeptide according to one of claims 1 to 3, for use in the treatment of pathologies of accidental, traumatic or degenerative type, including Alzheimer's disease, multiple sclerosis, Parkinson's disease, lesion, head trauma or lesions of the optic, olfactory, auditory nerves or of any cranial or peripheral nerve.
